# EUROPÄISCHE PATENTSCHRIFT

(11) **EP 1 279 396 B1**
(45) Veröffentlichungstag und Bekanntmachung des Hinweises auf die Patenterteilung: **26.09.2007**
(21) Anmeldenummer: 02090267.2
(22) Anmeldetag: 18.07.2002
(51) Int. Cl.: A61K 8/81, A61Q 19/00, A61Q 17/04, C08L 33/00

(54) **Kosmetisches Produkt mit Acrylaten**
Cosmetic product with acrylates
Produit cosmétique avec des acrylates

(30) Priorität: 24.07.2001 DE 10136882
(43) Veröffentlichungstag der Anmeldung: 29.01.2003
(73) Patentinhaber: Coty B.V., 2031 CC Haarlem (NL)
(72) Erfinder: Loginova, Yelena, Bronx, NY 10467 (US); Cernasov, Domnica, Ringwood, NJ 07456 (US); Macchio, Ralph, Sparta, NJ 07971 (US)
(74) Vertreter: Walter, Wolf-Jürgen

(56) Entgegenhaltungen:
- EP-A- 0 590 604
- FR-A- 2 787 322
- US-A- 3 639 572
- PATENT ABSTRACTS OF JAPAN Bd. 4, Nr. 16 (C-072), 7. Februar 1980 (1980-02-07) & JP 54 151139 A (SHISEIDO CO), 28. November 1979 (1979-11-28)

## Beschreibung

Die Erfindung betrifft ein kosmetisches Produkt mit Acrylaten sowie ein Verfahren zur Herstellung dieses Produktes.

Aus der US-A-5925337 ist eine wasserfeste Mascara-Zusammensetzung bekannt, die 2-40 Gew-% eines Wachses, 5-15 Gew-% eines Verdickungsmittels, 35-50 Gew-% eines flüchtigen organischen Lösungsmittels und 1-35 Gew-% eines wasserlöslichen filmbildenden Mittels enthält, wobei letzteres z.B. auch ein Acrylatpolymer sein kann. Die Zusammensetzung enthält keinen Emulgator.

Die FR 2787322-A beschreibt eine OW-Emulsion mit einem biologisch aktiven Mittel und einem Emulgiersystem mit Acrylates/C10-30 Alkylacrylate Crosspolymer.

Der Erfindung liegt die Aufgabe zugrunde, ein neues kosmetisches Zwischenprodukt bereitzustellen, das auf Grund seiner besonderen Struktur geeignet ist, ein weiches und elastisches Produkt mit wasserfesten Eigenschaften zu bilden, das sowohl mit auf Wasser basierenden Ingredienzen als auch auf organischen Lösungsmitteln basierenden Ingredienzen zu weiteren kosmetischen Produkten verarbeitet werden kann.

Erfindungsgemäß ist die kosmetische Zusammensetzung mit Acrylaten dadurch gekennzeichnet, daß sie enthält
(a) 0.01-80 Gew-% eines mit Wasser emulgierbaren, filmbildenden Mittels auf Basis eines Ethylacrylat/Methymethacrylat-Copolymern
(b) 0.01-90 Gew-% eines aliphatischen Kohlenwasserstoff-Lösungsmittels oder eines flüchtigen Siliconderivates, wobei beide mit Wasser nicht mischbar sind und mit auf Wasser basierenden Ingredienzen und/oder mit auf organischen Lösungsmitteln basierenden Ingredienzen in Gegenwart eines Emulgators emulgierbar sind;
(c) 0.01 - 5 Gew-% eines nicht-ionischen Emulgators;
(d) 1.00-80 Gew-% Wasser;
und wobei die Zusammensetzung die Struktur eines Gels hat und wobei alle Prozentangaben auf das Gesamtgewicht der Zusammensetzung bezogen sind.

Als Emulgator können vorteilhaft alkoxylierte Alkohole, ethoxylierte Alkohole, Polyglycerinester und Gemische davon verwendet werden. Dabei handelt es sich um nicht-ionische Emulgatoren, die als hydrophile Gruppe z.B. eine Polyolgruppe, eine Polyalkylglycolethergruppe oder eine Kombination davon enthält, wie z.B. Anlagerungsprodukte von 2-30 Mol Ethylenoxid an lineare Fettalkohole mit 8-22 Kohlenstoffatomen und an Fettsäuren mit 12-22 Kohlenstoffatomen. Weiterhin gehören dazu Polyglycerylester wie z.B. Polyglycerinricinoleat oder Polyglycerinpoly-12-hydroxystearat sowie Gemische dieser verschiedene Substanzklassen.

Ein bevorzugter Emulgator ist beispielsweise Laureth-20, Laureth-23, Oleth-20, Steareth-20, Steareth-50, Ceteareth-20, Ceteareth-30 oder Ceteareth-50.

Besonders bevorzugt wird, wenn das filmbildende Mittel ein Ethylacrylat/Methylmethacrylat-Copolymeres mit einem Verhältnis vom Ethylacrylat-Einheiten zu Methylmethacrylat-Einheiten in dem Polymeren im Bereich von 7,5-8,5 : 1,8-2,3 ist.

Ein bevorzugter Bereich für das Acrylatcopolymere liegt zwischen 0,1 und 50 Gew-%, insbesondere 0,1-15 Gew-%.

Als aliphatisches Kohlenwasserstoff-Lösungsmittel, das mit Wasser nicht mischbar ist, werden vorzugsweise Isoparaffine (z.B. Isododecan, das besonders bevorzugt ist), Pentan, Hexan, Decan usw. verwendet.

Ein bevorzugter Bereich für das aliphatische Kohlenwasserstoff-Lösungsmittel oder das flüchtige Siliconderivat liegt zwischen 30 und 75 Gew-%, insbesondere im Bereich 51-70 Gew-%.

Als flüchtiges Siliconderivat kann vorteilhaft Dimethicone oder Cyclomethicone eingesetzt werden.

Ein bevorzugtes Siliconderivat ist z.B. Decamethylcyclopentasiloxysiloxan (Silicone SS4230).

Der bevorzugte Anteil von Wasser liegt im Bereich von 1,0 bis 40 Gew-%, insbesondere 1,0 bis 20 Gew-%.

Das erfindungsgemäße Produkt ist ein Gel, das sich besonders dadurch auszeichnet, daß eine in der kosmetischen Formulierung stabile Kombination von Lösungsmittel und Filmbildner erhalten wird. Eine solche stabile Kombination mit Gelcharakter war für den Fachmann überraschend, und sie gestattet die Herstellung von kosmetischen Produkten mit besonders hoher Elastizität und Weichheit. So läßt sich beispielsweise die Elastizität auf das 4- bis 5-fache steigern gegenüber der Elastizität des eingesetzten Filmbildners, z.B. bei einem wäßrigen Ethylacrylat/Methylmethacrylat-Copolymer.

Die Elastizität wurde ermittelt durch die Teststreifen-Methode, die folgendermaßen durchgeführt wird:
Es wird die Länge eines aus dem Polymeren hergestellten elastischen Filmes gemessen. Auf eine nichtklebrigen Oberfläche werden separat zwei Filme A und B von etwa 150 µm (6,0 mil) Dicke mittels eines Bird-Applikators gegossen. Film A besteht aus einem wäßrigen Acrylatcopolymeren (Acrylates Copolymer :
Wasser etwa 50:50, Emulgator), Film B besteht aus 50 % Bestandteile A und 50 % Isododecane. Nach 24 Stunden bei 25 °C werden Probestreifen von etwa 50x25 mm aus beiden Filmen herausgeschnitten. Ein Ende des Films wird an einem Lineal eingespannt. Die Proben werden in Richtung auf das Linealende bis zum Abriß gezogen. Das Ergebnis für Film A betrug 101 mm, für Film B 406 mm.

Auch die Weichheit der Zusammensetzung nach dem Auftragen auf die Haut oder auf eine ebene Fläche zeigt sich deutlich erhöht gegenüber dem ursprünglichen Filmbildner. Dadurch verbessert sich das Hautgefühl (feeling), das eine wesentliche Rolle spielt bei der Produktwahl durch den Anwender.

Die Erfindung betrifft auch die Herstellung einer kosmetischen Zusammensetzung mit Acrylaten. Das Verfahren besteht darin, daß
a) ein Gemisch aus 0,1 - 80 Gew-% eines filmbildenden Mittels auf Basis eines Acrylatcopolymeren ausgewählt unter einem Ethylacrylat/Methylmethacrylat-Copolymern oder einem Copolymeren, hergestellt aus n-Butylacrylat, tert-Butylacrylat und Ethyl- oder Butylmethacrylat
   1,0-80 Gew-% Wasser und 0,01-1 Gew-% eines nicht-ionischen Emulgators auf eine Temperatur im Bereich von 45 bis 50 °C - erwärmt wird;
b) die erhaltene Emulsion bei dieser Temperatur mit 0,1-90 Gew-% eines flüchtigen aliphatischen Kohlenwasserstoff-Lösungsmittels oder eines flüchtigen Siliconderivates vermischt wird, wobei das Lösungsmittel oder das Siliconderivat mit Wasser nicht mischbar sind, jedoch mit auf Wasser basierenden Ingredienzen und/oder mit auf organischen Lösungsmitteln basierenden Ingredienzen in Gegenwart eines Emulgators emulgierbar sind, und wobei nach Homogenisierung bei 1500-3000 U/min für 15 bis 60 Minuten ein zweiphasiges flüssiges System erhalten wird bei vollständiger Verteilung der mit Wasser nicht mischbaren organischen Phase als Mikrotröpfchen in der wäßrigen Phase; und
c) das Gemisch auf 25-30 °C abgekühlt wird unter Rühren mit einer Geschwindigkeit von 300 bis 600 U/min.

Dabei kann die Emulsion von Stufe a) weitere kosmetische Bestandteile enthalten,wie Ester, öle, Sonnenschutzmittel, Parfüm, Konservierungsmittel, Vitamine, Radikalfänger (scavenger) und weitere kosmetische Hilf- und/oder Wirkstoffe. Derartige Stoffe können gegebenenfalls als separate Phase hergestellt und zu der Emulsion von Stufe a) zugegeben werden.

Gegenstand der Erfindung ist auch eine Kosmetische Zusammensetzung mit der Struktur eines Gels und erhältlich durch
- Erwärmen eines Gemisches aus 0,1 - 80 Gew-% eines filmbildenden Mittels auf Basis eines Acrylatcopolymeren, ausgewählt unter einem Ethylacrylat/Methylmethacrylat-Copol ern oder einem Copolymeren, hergestellt aus n-Butylacrylat, tert-Butylacrylat und Ethyl- oder Butylmethacrylat;
   1,0-80 Gew-% Wasser und 0,01-1 Gew-% eines nicht-ionischen Emulgators auf eine Temperatur im Bereich von 45 bis 50 °C
- Vermischen der erhaltenen Emulsion mit 0,1-90 Gew-% eines flüchtigen aliphatischen Kohlenwasserstoff-Lösungsmittels oder eines flüchtigen Siliconderivates, wobei beide mit Wasser nicht mischbar sind und mit auf Wasser basierenden Ingredienzen und/oder mit auf organischen Lösungsmitteln basierenden Ingredienzen in Gegenwart eines Emulgators emulgierbar sind, und wobei ein zweiphasiges flüssiges System erhalten wird nach Homogenisieren bei 1500 bis 3000 U/min für 15 bis 60 Minuten bis zur vollständigen Verteilung der mit Wasser nicht mischbaren organischen Phase als Mikrotröpfchen in der wässrigen Phase;
- Abkühlen des Gemisches auf 25-30 °C unter Rühren mit einer Geschwindigkeit von 300 bis 600 U/min; und
- gegebenenfalls Zusatz weiterer kosmetischer Hilfsstoffe, Trägerstoffe, Wirkstoffe oder Gemische davon,
wobei alle Prozentangaben auf das Gesamtgewicht der Zusammensetzung bezogen sind.

Das erfindungsgemäße Verfahren zeichnet sich durch eine genau vorgegebene Abfolge von Schritten sowie ein unübliches Temperaturregime aus. Nur auf diese Weise gelingt es, eine stabile Formulierung mit den oben genannten Vorteilen zu erhalten.

Die Erfindung soll nachstehend durch Beispiele näher erläutert werden, die jedoch keine Einschränkung der Erfindung darstellen. Alle Angaben sind in Gewichtsprozent.

### Beispiel 1 Basisgel

| | |
|---|---|
| Ethyl Acrylate/Methyl Methacrylate | 24,5 |
| Wasser | 25,0 |
| Laureth-20 | 0,3 |
| Isododecane | 50,0 |

Das Copolymer wird Wasser und dem Emulgator Laureth-20 vermischt und das Gemisch auf 45 °C erwärmt. Isododecan wird unter Rühren hinzugegeben und die Temperatur auf 50 °C erhöht. Danach erfolgt Homogenisieren mit 2200 U/min für eine Zeit von 18 Minuten in einem 1000 ml Laborbehälter. Dann wird das Ge-misch auf 27 °C abgekühlt und dabei mit 400 U/min etwa 5 Min gerührt.

### Beispiel 2 Sonnenschutzgel

| | |
|---|---|
| Basisgel von Beispiel 1 | 98 |
| octyl Methoxycinnamate | 2 |

Es wird wie im Beispiel 1 gearbeitet, wobei das Sonnenschutzmittel zusammen mit dem Copolymer, Wasser und dem Emulgator verrührt wird.

### Beispiel 3 Gel-Lotion

| | |
|---|---|
| Basisgel von Beispiel 1 | 80 |
| Öl oder Ester | 20 |

Es wird wie im Beispiel 2 gearbeitet.

## Patentansprüche

1. Kosmetische Zusammensetzung mit Acrylaten, **dadurch gekennzeichnet, dass** sie enthält
(a) 0,01-80 Gew-% eines mit Wasser emulgierbaren, filmbildenden Mittels auf Basis eines Ethylacrylat/Methylmethacrylat-Copolymern;
(b) 0,01-90 Gew-% eines aliphatischen Kohlenwasserstoff-Lösungsmittels oder eines flüchtigen Siliconderivates, wobei beide mit Wasser nicht mischbar sind und mit auf Wasser basierenden Ingredienzen und/oder auf organischen Lösungsmitteln basierenden Ingredienzen in Gegenwart eines Emulgators emulgierbar sind;
(c) 0,01 - 5 Gew-% eines nicht-ionischen Emulgators;
(d) 1-80 Gew-% Wasser;
und wobei die Zusammensetzung die Struktur eines Gels hat und wobei alle Prozentangaben auf das Gesamtgewicht der Zusammensetzung bezogen sind.

2. Zusammensetzung nach Anspruch 1, **dadurch gekennzeichnet, dass** der Emulgator aus der Gruppe ausgewählt ist, bestehend aus alkoxylierten Alkoholen, ethoxylierten Alkoholen, Polyglyceryl Estern und Gemischen davon.

3. Zusammensetzung nach Anspruch 1, **dadurch gekennzeichnet, dass** das filmbildende Mittel ein Ethylacrylat/Methylmethacrylat-Copolymeres mit einem Verhältnis vom Ethylacrylat-Einheiten zu Methylmethacrylat-Einheiten in dem Polymeren im Bereich von 7,5-8,5 : 1,8-2,3 ist.

4. Verfahren zur Herstellung einer kosmetischen Zusammensetzung mit Acrylaten, **gekennzeichnet durch** die folgenden Schritte:
- Erwärmen eines Gemisches aus 0,1 - 80 Gew-% eines filmbildenden Mittels auf Basis eines Acrylatcopolymeren, ausgewählt unter einem Ethylacrylat/Methylmethacrylat-Copolymern oder einem Copolymeren, hergestellt aus n-Butylacrylat, tert-Butylacrylat und Ethyl- oder Butylmethacrylat;
1,0-80 Gew-% Wasser und 0,01-1 Gew-% eines nicht-ionischen Emulgators auf eine Temperatur im Bereich von 45 bis 50 °C
- Vermischen der erhaltenen Emulsion mit 0,1-90 Gew-% eines flüchtigen aliphatischen Kohlenwasserstoff-Lösungsmittels oder eines flüchtigen Siliconderivates, wobei beide mit Wasser nicht mischbar sind und mit auf Wasser basierenden Ingredienzen und/oder mit auf organischen Lösungsmitteln basierenden Ingredienzen in Gegenwart eines Emulgators emulgierbar sind, und
wobei ein zweiphasiges flüssiges System erhalten wird nach Homogenisieren bei 1500 bis 3000 U/min für 15 bis 60 Minuten bis zur vollständigen Verteilung der mit Wasser nicht mischbaren organischen Phase als Mikrotröpfchen in der wässrigen Phase;
- Abkühlen des Gemisches auf 25-30 °C unter Rühren mit einer Geschwindigkeit von 300 bis 600 U/min.

5. Kosmetische Zusammensetzung mit der Struktur eines Gels und erhältlich durch
- Erwärmen eines Gemisches aus 0,1 - 80 Gew-% eines filmbildenden Mittels auf Basis eines Acrylatcopolymerenausgewählt unter einem Ethylacrylat/Methylmethacrylat-Copolymern oder einem Copolymeren, hergestellt aus n-Butylacrylat, tert-Butylacrylat und Ethyl- oder Butylmethacrylat; 1,0-80 Gew-% Wasser und 0,01-1 Gew-% eines nicht-ionischen Emulgators auf eine Temperatur im Bereich von 45 bis 50 °C
- Vermischen der erhaltenen Emulsion mit 0,1-90 Gew-% eines flüchtigen aliphatischen Kohlenwasserstoff-Lösungsmittels oder eines flüchtigen Siliconderivates, wobei beide mit Wasser nicht mischbar sind und mit auf Wasser basierenden Ingredienzen und/oder mit auf organischen Lösungsmitteln basierenden Ingredienzen in Gegenwart eines Emulgators emulgierbar sind, und
wobei ein zweiphasiges flüssiges System erhalten wird nach Homogenisieren bei 1500 bis 3000 U/min für 15 bis 60 Minuten bis zur vollständigen Verteilung der mit Wasser nicht mischbaren organischen Phase als Mikrotröpfchen in der wässrigen Phase;
- Abkühlen des Gemisches auf 25-30 °C unter Rühren mit einer Geschwindigkeit von 300 bis 600 U/min; und
- gegebenenfalls Zusatz weiterer kosmetischer Hilfsstoffe, Trägerstoffe, Wirkstoffe oder Gemische davon,
wobei alle Prozentangaben auf das Gesamtgewicht der Zusammensetzung bezogen sind.

## Claims

1. A cosmetic composition with acrylates **characterized in that** it contains
(a) 0.01-80 % by weight of a film-forming agent on the basis of an ethyl acrylate/methyl methacrylate copolymer being emulsifiable with water;
(b) 0.01-90 % by weight of an aliphatic hydrocarbon solvent or a volatile silicone derivate both of which being not miscible with water and being emulsifiable in the presence of an emulsifier with water-based ingredients and/or with ingredients on the basis of organic solvents;
(c) 0.01-5 % by weight of a non-ionic emulsifier;
(d) 1.00-80 % by weight of water;
and wherein the composition has the structure of a gel and wherein all percentages are based on the total weight of the composition.

2. A composition according to Claim 1 wherein the emulsifier is chosen from the group consisting of alcoxylated alcohols, ethoxylated alcohols, polyglyceryl esters and mixtures thereof.

3. A composition according to Claim 1 wherein the film-forming agent is an ethyl acrylate/methyl methacrylate copolymer in which the ratio of ethyl acrylate units to methyl methacrylate units in the polymer is in the range of 7.5-8.8 : 1.8-2.3.

4. A method for manufacturing a cosmetic composition with acrylates **characterized by** the following steps:
- heating up to a temperature in the range of 45 - 50 °C a mixture of 0.1-80 % by weight of a film-forming agent on the basis of an acrylate copolymer selected from an ethyl acrylate/methyl methacrylate copolymer or a copolymer prepared from n-butyl acrylate, tert-butyl acrylate and ethyl or butyl methacrylate, 1.0-80 % by weight of water and 0.01-1 % by weight of a non-ionic emulsifier,
- mixing the obtained emulsion with 0.1-90 % by weight of a volatile aliphatic hydrocarbon solvent or a volatile silicone derivate both of which being not miscible with water and being emulsifiable in the presence of an emulsifier with water-based ingredients and/or with ingredients on the basis of organic solvents, and wherein a two-phase liquid system is obtained after honogenization at 1500 - 3000 rpm during 15-60 min until the organic phase being not miscible with water is completely distributed in the aqueous phase in the form of micro-droplets;
- cooling down the mixture to 25-30 °C by stirring at a rate of 300 to 600 rpm.

5. Cosmetic composition with the structure of a gel and obtainable by
- heating up to a temperature in the range of 45 - 50 °C a mixture of 0.1-80 % by weight of a film-forming agent on the basis of an acrylate copolymer selected from an ethyl acrylate/methyl methacrylate copolymer or a copolymer prepared from n-butyl acrylate, tert-butyl acrylate and ethyl or butyl methacrylate, 1.0-80 % by weight of water and 0.01-1 % by weight of a non-ionic emulsifier,
- mixing the obtained emulsion with 0.1-90 % by weight of a volatile aliphatic hydrocarbon solvent or a volatile silicone derivate both of which being not miscible with water and being emulsifiable in the presence of an emulsifier with water-based ingredients and/or with ingredients on the basis of organic solvents, and wherein a two-phase liquid system is obtained after honogenization at 1500 - 3000 rpm during 15 - 60 min until the organic phase being not miscible with water is completely distributed in the aqueous phase in the form of micro-droplets;
- cooling down the mixture to 25-30 °C by stirring at a rate of 300 to 600 rpm and
- optionally adding further auxiliary cosmetic substances, carriers, active cosmetic substances or mixtures thereof,
wherein all percentages are based on the total weight of the composition.

## Revendications

1. Composition cosmétique avec des acrylates, **caractérisée en ce qu'**elle contient
(a) 0,01 à 80 % en poids d'un agent filmogène, émulsionnable avec d'eau, à base d'un copolymère éthylacrylate/méthylméthacrylate,
(b) 0,01 à 90 % en poids d'un solvant hydrocarbure aliphatique ou un d'un dérivé de silicone volatile, moyennant quoi les deux ne sont pas miscibles à l'eau et sont émulsionnables en présence d'un agent émulsifiant avec des ingrédients à base d'eau et/ou des ingrédients à base de solvants organiques,
(c) 0,01 à 5 % en poids d'un agent émulsifiant non ionique,
(d) 1 à 80 % d'eau,
et moyennant quoi la composition a la structure d'un gel et toutes les données indiquées en pourcentage se rapportent au poids total de la composition.

2. Composition selon la revendication 1, **caractérisée en ce que** l'agent émulsifiant est sélectionné dans le groupe constitué des alcools alkoxylés, des alcools éthoxylés, des esters de polyglycéryl et des mélanges d'entre eux.

3. Composition selon la revendication 1, **caractérisée en ce que** l'agent filmogène est un copolymère éthylacrylate/méthylméthacrylate ayant un rapport d'unités d'éthylacrylate aux unités de méthylméthacrylate dans le polymère dans une plage de 7,5 - 8,5 : 1,8 - 2,3.

4. Procédé de préparation d'une composition cosmétique avec des acrylates, **caractérisé par** les étapes suivantes :
- chauffage d'un mélange constitué de 0,1 à 80 % en poids d'un agent filmogène à base d'un copolymère d'acrylate, sélectionné parmi un copolymère éthylacrylate/méthylméthacrylate ou un copolymère préparé de n-butylacrylate, tert-butylacrylate et d'éthyl- ou de butylméthacrylate,
1,0 à 80 % en poids d'eau et 0,01 à 1 % en poids d'un agent émulsifiant non ionique, à une température dans une plage de 45 à 50°C,
- mélange de l'émulsion obtenue avec 0,1 à 90 % en poids d'un solvant hydrocarbure aliphatique volatile ou d'un dérivé de silicone volatile, moyennant quoi les deux ne sont pas miscibles à l'eau et sont émulsionnables en présence d'un agent émulsifiant avec des ingrédients à base d'eau et/ou des ingrédients à base de solvants organiques, et à l'occasion de quoi on obtient un système liquide à deux phases après homogénéisation à 1500 - 3000 t/min pendant 15 à 60 minutes jusqu'à dispersion complète de la phase organique non miscible à l'eau sous forme de microgouttelettes dans la phase aqueuse,
- refroidissement du mélange à 25-30°C sous agitation à une vitesse de 300 à 600 t/min.

5. Composition cosmétique ayant la structure d'un gel et pouvant être obtenue par
- chauffage d'un mélange constitué de 0,1 à 80 % en poids d'un agent filmogène à base d'un copolymère d'acrylate, sélectionné parmi un copolymère éthylacrylate/méthylméthacrylate ou un copolymère préparé de n-butylacrylate, tert-butylacrylate et d'éthyl- ou de butylméthacrylate,
1,0 à 80 % en poids d'eau et 0,01 à 1 % en poids d'un agent émulsifiant non ionique, à une température dans une plage de 45 à 50°C,
- mélange de l'émulsion obtenue avec 0,1 à 90 % en poids d'un solvant hydrocarbure aliphatique volatile ou d'un dérivé de silicone volatile, moyennant quoi les deux ne sont pas miscibles à l'eau et sont émulsionnables en présence d'un agent émulsifiant avec des ingrédients à base d'eau et/ou des ingrédients à base de solvants organiques, et à l'occasion de quoi on obtient un système liquide à deux phases après homogénéisation à 1500 - 3000 t/min pendant 15 à 60 minutes jusqu'à dispersion complète de la phase organique non miscible à l'eau sous forme de microgouttelettes dans la phase aqueuse,
- refroidissement du mélange à 25-30°C sous agitation à une vitesse de 300 à 600 t/min, et
- éventuellement ajout d'autres agents auxiliaires, substances de support ou principes actifs cosmétiques, ou de mélanges d'entre eux,
moyennant quoi toutes les données indiquées se rapportent au poids total de la composition.
